# EUROPEAN PATENT APPLICATION

(11) **EP 4 617 352 A1**
(43) Date of publication of application: **17.09.2025**
(21) Application number: 25163481.2
(22) Date of filing: 13.03.2025
(51) Int. Cl.: C12M 1/00, C12M 1/26

(54) **CELL DETACHMENT METHOD AND CELL DETACHMENT SYSTEM**

(30) Priority: 15.03.2024 JP 2024040945
(71) Applicant: CANON KABUSHIKI KAISHA, Tokyo 146-8501 (JP)
(72) Inventor: SURUGA, Eika, Tokyo (JP); NAKAMOTO, Atsushi, Tokyo (JP); YOSHIDA, Ryoichi, Tokyo (JP)
(74) Representative: WESER & Kollegen Patentanwälte PartmbB

(57) **Abstract**

A cell detachment method that detaches a cell sheet adhering to a culture surface of a culture container (8) from the culture container (8) by using a cell detachment system includes a tapping process of tapping the culture container (8) in a first direction that contains a direction component parallel to the culture surface; and a shaking process of shaking the culture container (8) in a second direction that contains a direction component parallel to the culture surface, in which the first direction and the second direction are different from each other as viewed in a direction perpendicular to the culture surface.

## Description

### BACKGROUND

### Field of the Disclosure

The present disclosure relates to a cell detachment method and a cell detachment system.

### Description of the Related Art

In the fields of regenerative medicine and cell medicine, recent years have seen attempts to culture cells in sheet forms and transplant the cultured sheet-shaped cells (cell sheets) to affected areas to repair damaged tissues, etc. When cell sheets are produced by using adherent cells, for example, cells are cultured into a sheet on a polystyrene dish, which is one example of a culture substrate, and are detached and recovered as a sheet from the culture substrate. The method for efficiently and stably producing cell sheets while reducing wrinkles, tears, holes, etc., in the cell sheets has been demanded.

Japanese Patent Laid-Open No. 2014-113133 discloses a cell detachment apparatus that includes a container holder for attaching a culture container to which cultured cells have adhered and a guide mechanism that guides back-and-forth movement of the container holder, in which the container holder is allowed to collide with a collision target member.

### SUMMARY OF THE DISCLOSURE

The present disclosure provides a cell detachment method that efficiently detaches a cell sheet while reducing tearing of the cell sheet. The present disclosure also provides a cell detachment system that efficiently detaches a cell sheet while reducing tearing of the cell sheet.

The present disclosure in its first aspect provides a cell detachment method as specified in claims 1 to 6.

The present disclosure in its second aspect provides a cell detachment system as specified in claim 7.

Further features of the present disclosure will become apparent from the following description of exemplary embodiments with reference to the attached drawings.

### BRIEF DESCRIPTION OF THE DRAWINGS

Fig. 1 is a schematic view of one example of a cell detachment system according to a first embodiment.
Fig. 2 is a cross-sectional view of a tapping unit according to the first embodiment.
Fig. 3 is a top schematic view illustrating the relationship between a tapping direction and a shaking direction according to the first embodiment.
Fig. 4 is a schematic view of one example of a cell detachment system according to the first embodiment.
Fig. 5 is a schematic view of one example of a cell detachment system according to the first embodiment.
Figs. 6A and 6B are each a schematic view of a uniaxial shaking mechanism according to the first embodiment.
Fig. 7 is a flowchart of a cell detachment method according to the first embodiment.

### DESCRIPTION OF THE EMBODIMENTS

When a cell sheet is being detached from a culture container that retains a liquid and has the cell sheet adhered thereto and when the direction in which the culture container is allowed to undergo back-and-forth movement is the same as the direction in which the impact is applied to the culture container, there has been instances where the cell sheet is prone to tearing. Under conditions employed for efficiently detaching a cell sheet in a shorter period of time, there has also been instances where the cell sheet is prone to tearing.

The present disclosure will now be described in detail through embodiments.

The inventors of the present disclosure have investigated a method for efficiently detaching a cell sheet while reducing tearing of the cell sheet. As a result, the inventors have found that a cell sheet can be efficiently detached by performing a tapping process of tapping a culture container to which a cell sheet adheres and a shaking process of shaking the culture container in combination.

Furthermore, the inventors have found that the cell sheet has tendency to start detaching from a position where an angle of a specified range is formed with respect to the direction in which the culture container is tapped.

Further studies have been conducted on the aforementioned findings, and it is found that a cell sheet can be more efficiently detached by shaking the culture container at an angle of a specified range with respect to the tapping direction. That is, it has been found that a cell sheet can be efficiently detached when the direction of tapping and the direction of shaking are different from each other as viewed in a direction perpendicular to the culture surface. The reasons why the cell sheet detachment efficiency is improved by the aforementioned features of the present disclosure while reducing tearing are presumably as follows.

The flow of the liquid generated by shaking the culture container enters the gap between the culture container and the cell sheet that has partly detached, and pushes up the cell sheet in a direction of further detaching the cell sheet. Here, when a water flow acts toward the portion where the cell sheet has partially detached, the cell sheet can be efficiently detached while reducing the tearing of the cell sheet.

### Cell detachment method

A cell detachment method according to a first embodiment detaches a cell sheet adhering to a culture surface of a culture container from the culture surface through a tapping process of tapping the culture container and a shaking process of shaking the culture container. Furthermore, in the cell detachment method of this embodiment, the tapping process involves tapping the culture container in a first direction containing a direction component parallel to the culture surface, and the shaking process involves shaking the culture container in a second direction containing a direction component parallel to the culture surface. A cell sheet can be efficiently detached while reducing tearing of the cell sheet when the first direction and the second direction are different from each other as viewed in a direction perpendicular to the culture surface.

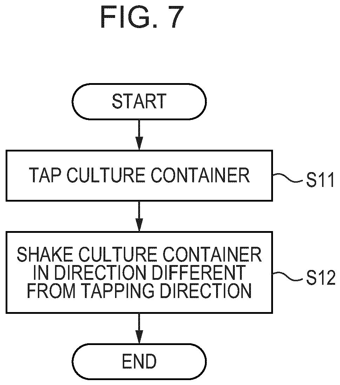

Fig. 7 is a flowchart of a cell detachment method according to this embodiment. First, in the tapping process (S11), the culture container to which the cell sheet adheres is tapped by a tapping unit. Next, in the shaking process (S12), the culture container is shaken in a direction different from the tapping by the tapping unit. The times at which the tapping process and the shaking process are started are not limited to this, and two process may be performed simultaneously for a some time period or may be performed alternatingly.

In this embodiment, the cell detachment method refers to a method for detaching a cell sheet adhering to the culture surface from the culture container by using external stimuli. Examples of the external stimuli include tapping, shaking, ultrasonic waves, discharging a liquid, and stirring a liquid. Discharging a liquid concerns a method of discharging a liquid from a needle or the like so that the liquid hits the culture surface, is scattered along the culture surface, and applies a shear force.

Stirring a liquid concerns a method of applying a shear force by forming a flow channel along the culture surface and feeding a liquid in one direction or by switching direction by using a pump or syringe. The cell detachment method may only involve external stimuli such as tapping and shaking, or may involve a combination of other external stimuli.

### Cell sheet

A cell sheet in this embodiment refers to a film in which cells are interconnected to form a sheet. The cells constituting the cell sheet are not particularly limited as long as the cells are able to form a cell sheet. Examples of the cells include adherend cells such as adherend body cells.

Examples of the body cells include myoblasts (for example, skeletal myoblasts), muscle satellite cells, and mesenchymal stem cells (for example, those derived from bone marrow, adipose tissue, peripheral blood, skin, hair roots, muscle tissue, endometrium, placenta, umbilical cord blood, etc.). Other examples include cardiomyocytes, fibroblasts, tissue stem cells such as cardiac stem cells, embryonic stem cells, pluripotent stem cells such as iPS cells, synovial cells, chondrocytes, and epithelial cells (for example, oral mucosa epithelial cells, retinal pigment epithelial cells, nasal mucosa epithelial cells, etc.)

Yet other examples include endothelial cells (for example, vascular endothelial cells, etc.), liver cells (for example, liver parenchymal cells, etc.), pancreatic cells (for example, pancreatic islet cells, etc.), kidney cells, adrenal cells, periodontal ligament cells, gingival cells, periosteal cells, and skin cells.

Moreover, the body cells may be those differentiated from iPS cells (iPS cell-derived cells). Examples of the iPS cell-derived cells include cardiomyocytes, fibroblasts, myoblasts, epithelial cells, endothelial cells, hepatic cells, pancreatic cells, kidney cells, adrenal cells, periodontal ligament cells, gingival cells, periosteal cells, skin cells, synovial cells, chondrocytes, etc., derived from iPS cells.

### Culture container

The culture container of this embodiment is not particularly limited as long as the culture container is a cell-adhering culture container. For example, the culture container is any one of flasks, tissue culture flasks, dishes, tissue culture dishes, multi-dishes, microplates, multi-well plates, multi-plates, culture bags, and bottles.

The material for the culture container of this embodiment may be any material that is chemically stable and that can culture desired cells. Examples of the material include polyethylene, polypropylene, polycarbonate, polystyrene, polyvinyl chloride, nylon, polyurethane, polyurea, polylactic acid, polyglycolic acid, polyvinyl alcohol, polyvinyl acetate, poly(meth)acrylic acid, poly(meth)acrylic acid derivatives, polyacrylonitrile, poly(meth)acrylamide, poly(meth)acrylamide derivatives, polysulfone, cellulose, cellulose derivatives, polysilicone, polymethylpentene, glass, and metal. Among these, polystyrene may be used from the viewpoint of stability.

Alternatively, a temperature responsive container that has a culture surface undergoing changes in hydrophilic properties by temperature may be used as the culture container of this embodiment.

### Cell remover

A cell remover in this description refers to a solution that is retained in the culture container when cells are detached by the cell detachment method according to the present disclosure. Here, the cell remover in this description does not have to contain a component that promotes detachment of the cell sheet. For example, from the viewpoint of protecting the state of the cell surfaces, a cell remover substantially free of proteolytic enzymes may be used.

Here, "substantially free of' means that the content is equal to or lower than 0.0005 mass%.

The pH of the cell remover in this embodiment may be in the neutral or acidic range.

The neutral range is suitable for cell culture and the survival rate of the cells can be stably maintained high. The pH can be adjusted, as appropriate, by using hydrochloric acid, sodium hydroxide, etc. In order to stabilize the pH, various buffer solutions may also be used.

Any buffer solution can be used without limitations in this embodiment as long as the neutral range can be maintained. Examples thereof include Tris buffer solutions such as Tris-HCl buffer solutions, phosphate buffer solutions, HEPES buffer solutions, citrate-phosphate buffer solutions, glycylglycine-sodium hydroxide buffer solutions, Britton-Robinson buffer solutions, and GTA buffer solutions. Among these, phosphate buffer solutions close to the in-vivo environment can be used, such as a phosphate buffered saline (PBS) obtained by adjusting a phosphate buffer solution adjusted to be isotonic with the intracellular fluid.

The viscosity of the cell remover in this embodiment can be 1.80 mPa·s or less. This is because flowing of the remover generated by ultrasonic vibrations is not obstructed, and the detachment efficiency can be maintained high. The viscosity of the cell remover can be adjusted, as appropriate, by adding a polymer, a saccharide, etc.

The cell remover of this embodiment may contain a proteolytic enzyme, but the amount of the proteolytic enzyme relative to the total mass of the cell remover can be 0.0005 mass% or less or zero. This is because a proteolytic enzyme possibly degrades the quality of the cells although the detachment efficiency can be increased as the proteolytic enzyme breaks down some part of the cells.

A proteolytic enzyme in this embodiment is an enzyme that breaks down some part of cells to facilitate detachment of the cells from the substrate. Examples thereof include trypsin, accutase, collagenase, natural proteases, chymotrypsin, elastase, papain, pronase, and recombinants thereof.

The cell remover in this embodiment may be a medium but is not particularly limited. A solution that contains a metal ion chelating agent (hereinafter may also be referred to as a chelating agent) may also be used. This is because using a cell remover containing a chelating agent can more effectively detach the cells by ultrasonic vibrations.

The chelating agent in this embodiment is not particularly limited. Examples of the chelating agent include ethylenediaminetetraacetic acid (hereinafter may also be referred to as EDTA), ethylenediamine, ethylenediaminetetramethylenephosphonic acid, glycol ether diaminetetraacetic acid, nitrilotriacetic acid, diethylenetriaminepentaacetic acid, iminodiacetic acid, dihydroxyethylglycine, dicarboxymethylglutamic acid, ethylenediaminedisuccinic acid, etidronic acid, citric acid, gluconic acid, and phosphonobutanetriacetic acid.

Among these, the chelating agent can form a chelate with a divalent cation, or can form a chelate with Ca2+ and Mg2+, and the chelating agent can be ethylenediaminetetraacetic acid. When ethylenediaminetetraacetic acid is used as the chelating agent, the pH of the cell remover may be 7.0 or more and 8.0 or less. This is because this level is rather high in the neutral range where the cell survival rate can be maintained high, and thus the chelating ability of ethylenediaminetetraacetic acid can be further enhanced and the detachment efficiency can be further enhanced.

The chelating agents can be used alone or in combination. The chelating agent content can be 0.01 mM or more and 5.0 mM or less. Within this range, the chelating effect can be securely obtained, and the decrease in activity caused by the presence of excessive chelating agents can also be reduced.

The cell remover of this embodiment may contain a hydrophilic polymer including a polyalkylene glycol structure. Polyalkylene glycol can increase the survival rate of the cells in the cell detachment method using ultrasonic waves. An example of the hydrophilic polymer including a polyalkylene glycol structure is polyethylene glycol. The hydrophilic polymer can have a peak molecular weight Mp of 800 or more and 50000 or less or can have a peak molecular weight Mp of 1200 or more and 20000 or less as measured by gel permeation chromatography. This is because the influence of the polymer on the cells is small, and the medium thickening effect caused by the polymer can be reduced.

The type of the culture medium may be any, and examples thereof include a Dulbecco's Modified Eagles's Medium (DMEM), Ham's Nutrient Mixture F12, a DMEM/F12 medium, a McCoy's 5A medium, an Eagles's Minimum Essential Medium (EMEM), α Modified Eagles's Minimum Essential Medium (αMEM), a Minimum Essential Medium (MEM), an RPMI-1640 medium, an Iscove's Modified Dulbecco's Medium (IMDM), an MCDB 131 medium, a William's E medium, an IPL-41 medium, a Fischer's medium, StemSpan H3000, StemSpan SFEM, Stemline II, Endothelial Cell Growth Medium 2 Kit, Mesenchymal Stem Cell Growth Medium 2, MSCGM Bullet Kit, mTeSR1, an mTeSR2 medium, Repro FF, Repro FF2, a NutriStem medium, and an MF-Medium Mesenchymal Stem Cell Growth Medium.

A medium that is suitable for culturing each cell type may be used among these.

A serum or an antibiotic may be added the aforementioned medium. Examples of the serum include fetal bovine serum (FBS), calf serum, adult bovine serum, horse serum, sheep serum, goat serum, pig serum, chicken serum, rabbit serum, and human serum, and in general, FBS is frequently used for its high availability. Alternatively, a serum-free medium that does not contain any of untreated or unpurified serums but contains purified blood-derived components or animal tissue-derived components (growth factors, etc.) may be used.

Examples of the antibiotic added to the medium include penicillin, streptomycin, ampicillin, carbenicillin, tetracycline, bleomycin, actinomycin, kanamycin, actinomycin D, and amphotericin B.

### Cell sheet culture conditions

The cell sheet culture conditions can be selected as appropriate for the cells to be cultured. Typically, an appropriate medium is added to a dish, about 1.0 × 10¹ to 1.0 × 10⁵ cells/cm² of cells are seeded therein, and culture is carried out in an environment at a temperature of 37°C and a CO₂ concentration of 5%. Here, culture may be carried out until the cell-occupied area ratio in the substrate is about 100%, in other words, until a confluent state is reached.

### Cell detachment system

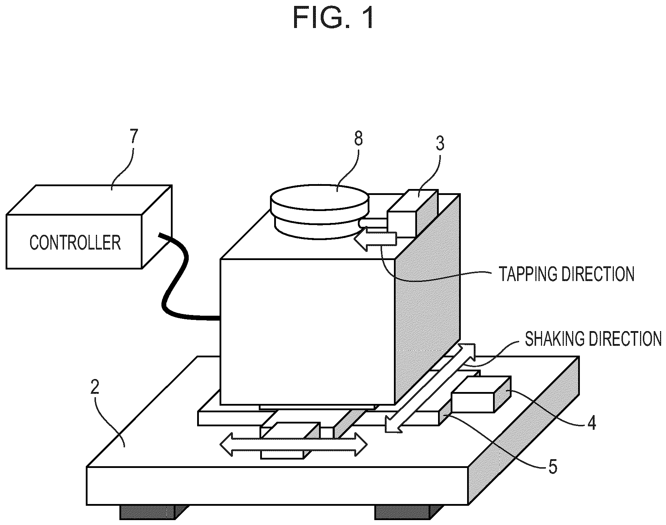

Fig. 1 is a schematic view of one example of a cell detachment system that performs the cell detachment method according to this embodiment. The culture container 8 is tapped by the tapping unit 3. Furthermore, the culture container 8 is shaken by the shaking unit 4. The cell detachment system according to this embodiment may have a function of applying external stimuli, such as ultrasonic vibrations.

### Tapping process

The tapping process in this embodiment includes a process of applying a repulsive force to the culture substrate in order to detach at least part of the cells adhering to the culture surface of the culture substrate from the culture surface. The tapping process is, for example, a process of performing at least one of a process of applying a repulsive force to the culture substrate by tapping the culture substrate itself, and a process of applying a repulsive force to the culture substrate by moving the culture substrate and causing the culture substrate to collide with a member that constitutes a cell detachment apparatus. The tapping process in this embodiment may be periodic or non-periodic, and the magnitude of the repulsive force applied to the culture substrate in the tapping process may be set appropriately. The tapping process in this embodiment may be continuously performed from the start to the completion of the cell detachment, may be terminated before completion, or may be performed intermittently.

The time at which tapping is performed in the tapping process may be set periodically or non-periodically. Tapping can be performed at periodic timings from the viewpoint of facilitating the start of detachment due to continuously applied impact to the entire container. The time and period of tapping may be changed with the passage of time after starting the operation of the cell detachment system.

The frequency of tapping in the tapping process of this embodiment means the period of one cycle taken for the state to return to the state of a particular time point. The frequency is indicated by f (Hz) and is the number of vibrations per second. The frequency may be indicated by the unit rpm as the number of rotations per minute. This means that, mainly for a rotating machine, one cycle is deemed as one regression, and the number of the same regression repeated in one minute is assumed as the frequency. The frequency is not particularly limited, and, in this embodiment, can be 0.1 Hz to 20 Hz or can be 0.1 Hz to 10 Hz. The magnitude relationship between the frequency ranges of the tapping process and the shaking process in this embodiment is not particularly limited, but the frequency of tapping process/frequency of shaking process ratio can be 1 to 50 and more or can be 1 to 15. The frequency of the tapping process may change with the passage of time.

The time for which impact is applied continuously and/or intermittently in the tapping process can be set, as appropriate, according to the cell properties of the cell sheet, the environment temperature, the type of the remover, and the detachment conditions. The position of tapping in the tapping process is not particularly limited; however, impact can be applied to the culture container or a holder and including being applied to a side surface of the culture container.

The tapping direction (first direction) in the tapping process is not particularly limited but may contain a direction component parallel to the culture surface. In particular, when impact is applied to the culture container or the holder, it becomes possible to generate an inertia force or a flow of the culture solution. The cell sheet can be detached by applying the aforementioned force. The direction of impact may be changed with the passage of time after starting the operation of the cell detachment system.

The environment temperature is not particularly limited and may be 20°C to 40°C from the viewpoint of maintaining the cell survival rate. When the temperature is close to the temperature during culture, the influence of the changes in temperature on the cells can be reduced, and the survival rate can be maintained high.

### Tapping unit

The tapping unit in this embodiment may be any feature that can carry out the aforementioned tapping process. The tapping unit includes, for example, an object to be collided against the culture substrate and a moving unit that moves this object to collide against the culture substrate, or includes a moving unit that moves the culture substrate to collide with a member constituting the cell detachment apparatus. The object may have a mass that can apply an appropriate repulsive force to the culture substrate without tearing the culture substrate. Examples of the shape of the object include a rod shape, a hammer shape, and a spherical shape. Examples of the moving unit include motors and solenoids that can generate a moving force that moves the object or the culture substrate through electrification. Here, the object or the culture substrate may be moved by using only the motors, solenoids, etc., or the motors and solenoids may be used in combination with a member, such as a spring member, that can store energy.

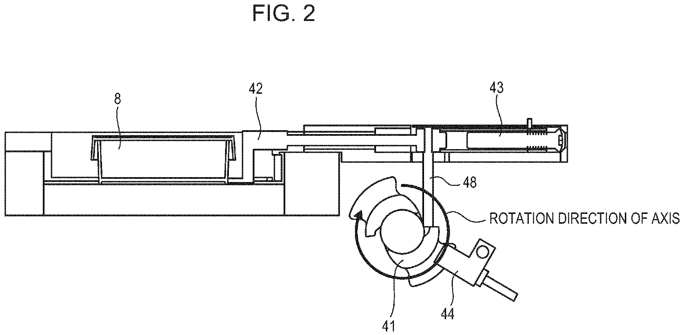

The tapping unit 3 starts detachment of the cell sheet by tapping the culture container 8. Fig. 2 is a cross-sectional view of one example of the tapping unit of this embodiment. A cam 41 is connected to a motor, and rotates in the rotation direction of the axis when driven by the motor. A hammer 42 is in contact with the cam 41 at a shaft 48, and is urged in a direction of tapping a culture container 8 by a spring 43. After the spring 43 is compressed by the rotation of the cam 41, the hammer 42 moves in such a direction that the profile of the cam 41 taps the culture container 8. The frequency of tapping can be determined by the rotation rate of the motor, and can be detected with a photocoupler 44.

### Shaking process

In this embodiment, the shaking process refers to a process in which the culture container or the portion that holds the culture container is moved. In the shaking process, the culture container is shaken to detach the cell sheet from the culture container.

The driving force of the shaking process is not limited to a spring and may include a magnet, an electromagnet, a motor, or the like.

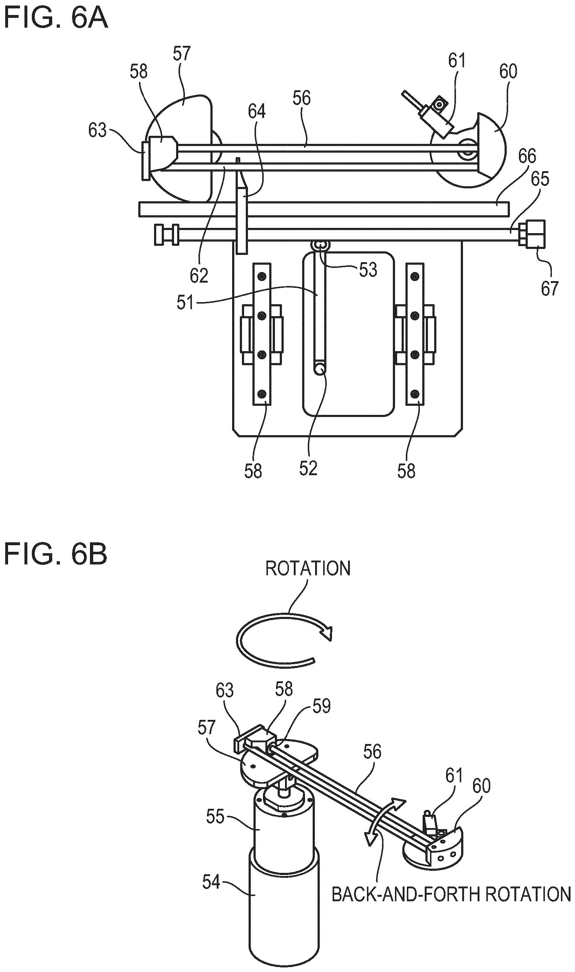

Figs. 6A and 6B illustrate a uniaxial shaking mechanism according to this embodiment. Guide rails 50 are fastened to a base plate 2 illustrated in Fig. 1 so as to shake the culture container 8 back and forth. A base-side spring post 52 to which a pressure spring 51 is connected is fixed to the base plate 2, and the pressure spring 51 is connected to the base-side spring post 52. The opposite end portion of the pressure spring 51 is fixed to a shaken-side spring post 53 provided in a shaken portion. The pressure spring 51 can be selected as necessary and has a detachable structure.

Shaking occurs as rotations of a shake motor 54 are transmitted to a shake rod 56 through a gear 55. A rotation disk 57 is fixed to the rotation axis of the shake motor 54 and rotates as illustrated in the drawing.

The rotation direction may be reversed. A linear bush holder 58 is rotatably installed at a position offset from the rotation axis, a linear bush 59 is built therein, and the shake rod 56 is built therein to be linearly movable. One end of the shake rod 56 is fixed to a rotatable shake fulcrum shaft 60. This translates movement into back-and-forth movement centered on the shake fulcrum shaft 60. The frequency of the back-and-forth movement is detected with a rotation photocoupler 61.

A connecting rod 62 is fastened to the shake rod 56 with a connecting rod fixing tool 63 so as to be parallel to the shake rod 56, and one end is fixed to the shake fulcrum shaft 60. A shake slide piece 64 equipped with the base plate 2 contacts the connecting rod 62 moving back and forth to generate back-and-forth shaking movement. The shake slide piece 64 is supported by a piece slide shaft 66 parallel to a shake adjust screw rod 65, and the shaking strokes can be freely adjusted by rotating the shake adjust screw rod 65. A stroke tab 67 is installed at an end of the shake adjust screw rod 65, and can be manually rotated to perform adjustment.

Fig. 1 is a schematic diagram of one example of the shaking structure according to this embodiment. Multiaxis rails 5 orthogonal to each other are biaxially arranged so that the direction of the shaking process can be freely changed. The multilaxis rails 5 are controlled by the controller 7, and the angle between the direction of tapping and the direction of shaking can be adjusted.

The operation of shaking in the shaking process can be set periodically or non-periodically. Shaking in the shaking process can be performed periodically from the viewpoint of facilitating the progress of detachment due to continuously applied physical impact to the entire culture container. The time and period of shaking may be changed with the passage of time after starting the operation of the cell detachment system.

The frequency of shaking in the shaking process of this embodiment means the period of one cycle taken for the state to return to the state of a particular time point. The frequency is indicated by f (Hz) and is the number of vibrations per second. The frequency may be indicated by the unit rpm as the number of rotations per minute. This means that, mainly for a rotating machine, one cycle is deemed as one regression, and the number of the same regression repeated in one minute is assumed as the frequency. The frequency is not particularly limited, and, in this embodiment, can be 0.1 Hz to 20 Hz or can be 0.1 Hz to 10 Hz. The frequency of shaking can be smaller than the frequency of tapping the culture container in the tapping process. The frequency of the shaking by the shaking unit may be changed with the passage of time after starting the operation of the cell detachment system.

The displacement of the position of the culture container in the shaking process can be 0.1 mm to 300 mm or can be 0.1 mm to 150 mm, but is not particularly limited. The displacement of the position of the culture container may be changed with the passage of time after starting the operation of the cell detachment system.

The time for which shaking is carried out in the shaking process can be set, as appropriate, according to the cell properties of the cell sheet, the environment temperature, the type of the remover, and the detachment conditions.

The shaking direction (second direction) in the shaking process is not particularly limited but may contain a direction component parallel to the culture surface in this embodiment. A flow along the culture surface can be generated inside the culture container, and thus the cell sheet can be effectively detached. The shaking direction in the shaking process may be changed with the passage of time after starting the operation of the cell detachment system.

The speed of shaking is not particularly limited, but when the speed at the side surface of the dish is measured, the speed can be 0.1 m/s to 1.0 m/s. The speed in the shaking process may be changed with the passage of time after starting the operation of the cell detachment system.

The environment temperature is not particularly limited but can be 20°C to 40°C or can be 37°C from the viewpoint of maintaining the cell survival rate. When the cells are handled at a temperature close to the temperature of culture, the influence of the changes in temperature on the cells can be reduced, and the survival rate can be maintained high.

### Angle formed between tapping process and shaking process

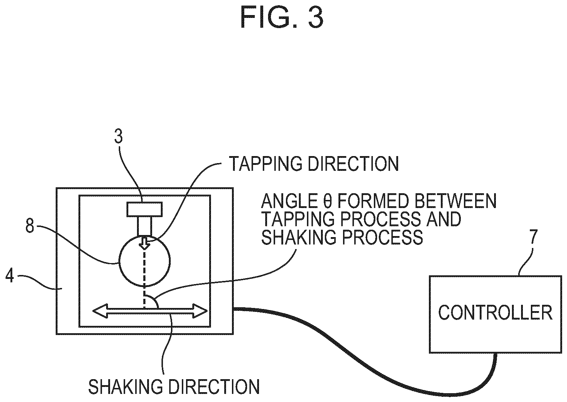

Fig. 3 indicates the relationship between the tapping direction in the tapping process and the shaking direction in the shaking process. The angle formed between the tapping direction and the shaking direction is the angle θ formed between the tapping direction and the shaking direction as viewed in a direction perpendicular to the culture surface. The directions of tapping and shaking may be changed with the passage of time.

The inventors of the present disclosure have found that the cell sheet has a tendency to start detaching from a position at an angle of a particular range with respect to the tapping direction. Here, the angle of a particular range is an angle of 45° or more and 135° or less. Furthermore, the inventors of the present disclosure have found that it is effective to generate a water flow countering the position where the cell sheet has started to detach for efficiently detaching the cell sheet while reducing tearing of the cells.

**In** other words, the tapping direction and the shaking direction can be different from each other, and can form an angle θ of 30° or more and 150° or less, or can form an angle θ of 45° or more and 135° or less.

### Vibrations in ultrasonic band

The cell detachment method according to this embodiment may include, in addition to the shaking process and the tapping process, an ultrasonic wave generating process of applying vibrations in the ultrasonic band to the culture container.

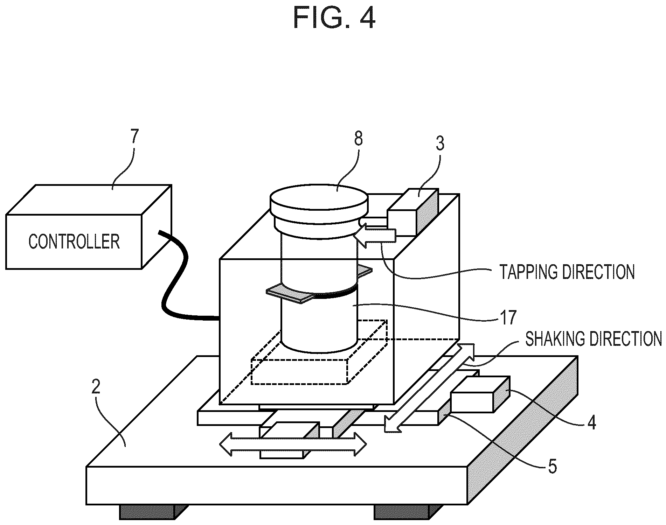

Fig. 4 is a schematic view of one example of a structure in which a mechanism that applies vertical vibrations to the culture container 8 is added to and combined with the cell detachment system of this embodiment. An ultrasonic wave generating unit can be used as the mechanism that applies vertical vibrations. An ultrasonic element 17 serving as the ultrasonic wave generating unit is disposed below the culture container 8 and vertically applies ultrasonic vibrations to the culture container 8. The ultrasonic element 17 is controlled by the controller 11, and the drive timing can be freely controlled.

Examples of the vibrations in the ultrasonic band are vibrations having frequencies of 10 kHz or more and 1 MHz or less. The vibration generating unit may be any feature that can apply vibrations in the ultrasonic band to the cells. On example is where an ultrasonic oscillator such as lead zirconium titanate (PZT) is used as a vibrating body.

The ultrasonic vibrating body may be any body that can generate ultrasonic waves, and an example thereof is a piezoelectric body and a vibrating plate bonded together. When the piezoelectric body has a circular shape, the vibrating plate may be glass, SUS, or quartz. When the vibrating plate is glass, SUS, or quartz, large amplitudes can be output at a relatively high driving frequency (vibration frequency) in the ultrasonic wave region without breaking the ultrasonic vibrating body.

In the case of a ring-shaped piezoelectric body, the outer diameter of the vibrating plate may be equal to the outer diameter of the piezoelectric body. The thickness of the vibrating plate may be such that, when the vibrating plate and the piezoelectric body are bonded together and undergo deflection vibrations, the midpoint of the deflection in the thickness direction, that is, the neutral plane that does not undergo either tension or contraction during deflection, may be on the vibrating plate side since the strain in the piezoelectric body can be highly efficiently used in the deflection.

Furthermore, a commercially available Langevin oscillator or rectangular oscillator can also be used as the ultrasonic vibrating body of the present disclosure. An example of the Langevin oscillator is an oscillator in which a piezoelectric body is interposed between two metal blocks and clamped together to form an integrated structure with bolts or the like.

### Time of applying external stimuli

The timing at which the external stimuli other than the tapping process and the shaking process start is not particularly limited.

The order in which the tapping, shaking, and external stimuli such as ultrasonic waves are started may be any. When ultrasonic stimuli are to be applied, the adhering force of the cells can be weakened while applying a shear force, and thus the cells can be more effectively detached when the tapping process and the shaking process are performed during or after application of stimuli such as ultrasonic waves. In order to determine transition to the external stimuli, the information regarding the cells adhering to the substrate may be measured and used.

In addition, other processes may be included. Examples of the other processes include a process of substituting the cell remover, a process of rinsing the cells with the cell remover, and a process of diluting and homogenizing the remover.

The aforementioned processes may be repeated at particular periods or irregular periods. The timing may be made uniform or may be adjusted for each process.

The ratio of the total time for which the tapping process and the shaking process are performed to the total time of the processes of external stimuli, such as ultrasonic waves, can be 0.01 or more and 100 or less.

### Process of changing shaking and tapping directions

The cell detachment method according to this embodiment may include, in addition to the shaking process and the tapping process, a process of changing the tapping direction and the shaking direction.

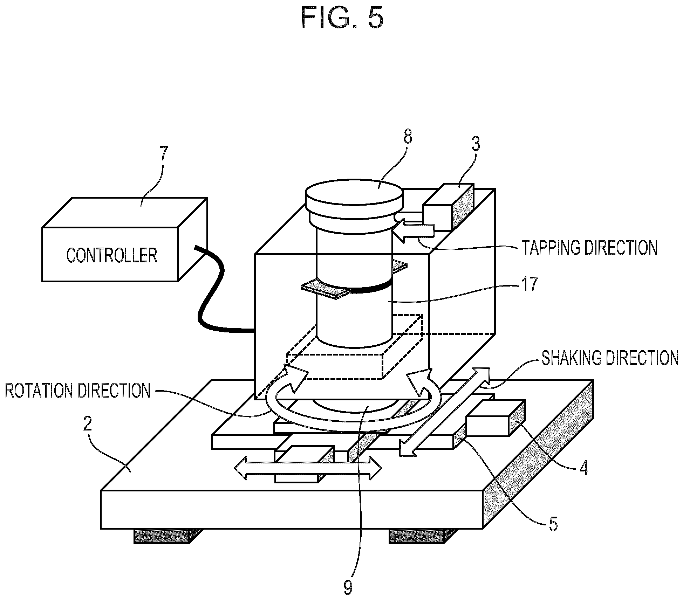

For example, since detachment starts by applying the detachment start unit, the detachment progressing direction can be changed by changing the direction in which the detachment start unit is applied to the culture container. Fig. 5 is a schematic diagram of one example of a mechanism that changes the tapping direction in this embodiment. A tapping unit 3 is configured such that the angle at which the culture container 8 is tapped can be freely changed, and the tapping direction can be freely changed due to this structure. The changing the tapping direction of the tapping unit 3 is controlled by the controller 7, and the shaking direction and the detachment progressing direction can be made substantially parallel to each other by changing the tapping direction. The tapping and shaking directions can be adjusted by the controller with the passage of time. The direction changing unit 9 may be a unit for changing the angle formed by the tapping direction by the tapping unit 3 and the shaking direction by the shaking unit 5, or may be a unit for changing the tapping direction with respect to the culture vessel 8. The direction changing unit 9 may be, for example, a rotatable mounting table.

In another example, in the cell detachment system illustrated in Fig. 1, the shaking direction is biaxially controlled, and thus the shaking direction can be changed to any angle within the operation plane. By changing the shaking direction, the shaking direction and the detachment progressing direction can be made substantially parallel to each other.

### Examples

The present disclosure will now be described in further detail through examples and comparative examples; however, the present disclosure is not limited in any way by the examples below without departing from the gist of the present disclosure.

### Culture of C2C12 cells on substrate

C2C12 cells, which are mouse myoblast cells, were seeded in a Φ35 temperature responsive dish (UpCell (registered trademark) produced by CellSeed Inc.) at a density of 65,000 cells/cm² and cultured in an environment of 37°C and a CO₂ concentration of 5%. The medium used was a DMEM/F12 medium (produced by Thermo Fisher Scientific Inc.) to which 10% of fetal bovine serum (produced by Sigma-Aldrich Co. LLC) and 1% of penicillin-streptomycin (10,000 U/ml produced by Thermo Fisher Scientific Inc.) were added. Culture was carried out for 2 days, and the state of the cells was observed with a phase contrast microscope to confirm cell adherence and growth. The cell-occupied area ratio of the dish was about 100%.

### Culture of A549 cells on substrate

A549 cells, which are human lung epithelial adenocarcinoma cells, were seeded in a Φ35 temperature responsive dish (UpCell (registered trademark) produced by CellSeed Inc.) at a density of 65,000 cells/cm² and cultured in an environment of 37°C and a CO₂ concentration of 5%. The medium used was DMEM (produced by Thermo Fisher Scientific Inc.) to which 10% of fetal bovine serum (produced by Sigma-Aldrich Co. LLC) and 1% of penicillin-streptomycin (10,000 U/ml produced by Thermo Fisher Scientific Inc.) were added. Culture was carried out for 7 days, and the state of the cells was observed with a phase contrast microscope to confirm cell adherence and growth. The cell-occupied area ratio of the dish was about 100%.

### Culture of BAEC cells on substrate

BAEC cells, which are bovine aorticendothelial cells, were seeded in a Φ35 polystyrene dish (produced by Corning Incorporated) at a density of 20,000 cells/cm² and cultured in an environment of 37°C and a CO₂ concentration of 5%. The medium used was DMEM (produced by Thermo Fisher Scientific Inc.) to which 10% of fetal bovine serum (produced by Sigma-Aldrich Co. LLC) and 1% of penicillin-streptomycin (10,000 U/ml produced by Thermo Fisher Scientific Inc.) were added. Culture was carried out for 7 days, and the state of the cells was observed with a phase contrast microscope to confirm cell adherence and growth. The cell-occupied area ratio of the dish was about 100%.

### Culture of HEK293 cells on substrate

HEK293 cells, which are human embryonic kidney cells, were seeded in a Φ35 temperature responsive dish (UpCell (registered trademark) produced by CellSeed Inc.) at a density of 65,000 cells/cm² and cultured in an environment of 37°C and a CO₂ concentration of 5%. The medium used was an Eagle's MEM medium (produced by FUJIFILM Wako Pure Chemical Corporation) to which 10% of fetal bovine serum (produced by Sigma-Aldrich Co. LLC) and 1% of penicillin-streptomycin (10,000 U/ml produced by Thermo Fisher Scientific Inc.) were added. Culture was carried out for 9 days, and the state of the cells was observed with a phase contrast microscope to confirm cell adherence and growth. The cell-occupied area ratio of the dish was about 100%.

### Culture of HUVEC cells on substrate

HUVEC cells, which are human umbilical vein endothelial cells, were seeded in a Φ35 temperature responsive dish (UpCell (registered trademark) produced by CellSeed Inc.) at a density of 65,000 cells/cm² and cultured in an environment of 37°C and a CO₂ concentration of 5%. The medium used was Endothelial Cell Growth Medium 2 Kit(produced by PromoCell GmbH) to which 10% of fetal bovine serum (produced by Sigma-Aldrich Co. LLC) and 1% of penicillin-streptomycin (10,000 U/ml produced by Thermo Fisher Scientific Inc.) were added. Culture was carried out for 7 days, and the state of the cells was observed with a phase contrast microscope to confirm cell adherence and growth. The cell-occupied area ratio of the dish was about 100%.

### Culture of MDCK cells on substrate

MDCK cells, which are Madin-Darby canine kidney cells, were seeded in a Φ35 temperature responsive dish (UpCell (registered trademark) produced by CellSeed Inc.) at a density of 65,000 cells/cm² and cultured in an environment of 37°C and a CO₂ concentration of 5%. The medium used was an Eagle's MEM medium (produced by FUJIFILM Wako Pure Chemical Corporation) to which 10% of fetal bovine serum (produced by Sigma-Aldrich Co. LLC) and 1% of penicillin-streptomycin (10,000 U/ml produced by Thermo Fisher Scientific Inc.) were added. Culture was carried out for 8 days, and the state of the cells was observed with a phase contrast microscope to confirm cell adherence and growth. The cell-occupied area ratio of the dish was about 100%.

### Culture of hMSC cells on substrate

hMSC cells, which are human mesenchymal stem cells, were seeded in a Φ35 temperature responsive dish (UpCell (registered trademark) produced by CellSeed Inc.) at a density of 30,000 cells/cm² and cultured in an environment of 37°C and a CO₂ concentration of 5%. The medium used was an Mesenchymal Stem Cell Growth Medium 2 (produced by PromoCell GmbH) to which 10% of fetal bovine serum (produced by Sigma-Aldrich Co. LLC) and 1% of penicillin-streptomycin (10,000 U/ml produced by Thermo Fisher Scientific Inc.) were added. Culture was carried out for 7 days, and the state of the cells was observed with a phase contrast microscope to confirm cell adherence and growth. The cell-occupied area ratio of the dish was about 100%.

### Driving method

A cell sheet adhering to the culture container was detached by driving the tapping unit 3, the shaking unit 5, and the ultrasonic wave generating unit by using the cell detachment system illustrated in Fig. 5 at an environment temperature of 37°C.

Additional vertical vibrations: ultrasonic vibrations

A dish was set from above onto an apparatus having a Langevin element illustrated in Fig. 5 built therein. A Langevin oscillator having a resonance frequency of 36 kHz was used as the ultrasonic element, and ultrasonic waves were continuously applied from the bottom at a frequency of 36 kHz and an input voltage of 20 V.

### Evaluation of detachability

The detachability was evaluated from two items: the quality evaluation of the cell sheet in a detached state and the detachment time. The detached state of the cell sheet in each example means the state that includes tearing of the cell sheet after detached from the culture surface. The quality of the cell sheet in each example was evaluated by observing the cell sheet in a detached state. Moreover, the detachment time in each example means the time taken for the cell sheet to detach from the culture surface. In the examples, the state in which the cell sheet has detached means that the entire surface of the cell sheet has floated upward from the culture surface of the culture container. Since the time required for detachment differs for each cell type, the detachment time was evaluated by determining the ratio of the detachment time of Example to the detachment time of Comparative Example for the same cell type. The evaluation was carried out by observation using naked eye, a camera, a phase contrast microscope, a fluorescence microscope using a staining liquid, etc. The quality and the detachment time of the cell sheet were evaluated on the basis of the following standards, and a rating of C or higher was determined to have an effect of the present disclosure.

### Quality evaluation of cell sheet

A: No holes or tears were generated.
B: At least one of a hole and a tear smaller than 500 µm was generated.
C: At least one of a hole and a tear 500 µm or larger but smaller than 1 mm was generated.
D: At least one of a hole and a tear 1 mm or larger was generated.

### Evaluation of detachment time

AA: The time was shortened by 30% or more.
A: The time was shortened by 20% or more and less than 30%.
B: The time was shortened by 10% or more and less than 20%.
C: The time was shortened by 1% or more and less than 10%.
D: The detachment time was as long as that in Comparative Examples.

### Example 1

Detachment of a cell sheet of C2C12 cells on the culture container was studied as follows. A cell sheet of C2C12 cells was cultured under the aforementioned culture conditions. After confirming formation of a cell sheet, the culture solution in the culture container was replaced with a fresh medium 4 hours before studying the detachment, and the culture was continued in a 37°C incubator. Then the culture container was taken out of the 37°C incubator and set in the detachment system illustrated in Fig. 5, and the system was driven under the aforementioned driving conditions using the conditions indicated in Table 1. In this example, the tapping unit 3 and the shaking unit 5 were used but the ultrasonic wave generating unit 8 was not used for driving.

The angle θ between the tapping direction and the shaking direction was changed by controlling a rotating stage that changes the tapping direction according to the changing unit 1 described above. In this example, the angle θ was set to 90°.

Driving was performed under these conditions, and the detachability was evaluated after the cell sheet was detached. The quality of the cell sheet was evaluated as A. The detachment time in comparison with the result of Comparative Example 1 under the same cell type conditions was rated A. Since all of the evaluation items were rated C or higher, it was determined that an effect of the present disclosure was exhibited.

### Examples 2 to 17

The detachment of the cell sheet was studied as in Example 1 except that the combinations of the cell type and the driving conditions of the mechanisms were changed as indicated in Table 1. The aforementioned ultrasonic wave generating conditions were used as the ultrasonic wave generating unit used in applying additional vibrations in Example 11.

The detachability of each example was evaluated by respectively comparing with the conditions of Comparative Examples 1 to 7 that used the same cell type conditions. The results are indicated in Table 2. In all of Examples, the results were rated C or higher, and it was determined that an effect of the present disclosure was exhibited.

### Comparative Examples 1 to 7

The detachment of the cell sheet was studied as in Example 1 except that the combinations of the cell type of the cell sheet and the driving conditions of the mechanisms were changed as indicated in Table 1. The quality of the cell sheet was evaluated as D in all of the comparative examples. Furthermore, the detachment time was 9 minutes for C2C12, 44 minutes for A549, 33 minutes for HEK293, 30 minutes for HUVEC, 90 minutes for MDCK, 43 minutes for hMSC, and 102 minutes for BAEC, and was rated D.

**Table 1: Process conditions of Examples 1 to 17 and Comparative Examples 1 to 7**

| | Cell type | Culture container | Angle θ (°) between tapping and shaking directions | Tapping spring force (N) | Tapping frequency (Hz) | Shaking frequency (Hz) | Additional vibrations |
|---|---|---|---|---|---|---|---|
| Example 1 | C2C12 | Temperature responsive dish | 90 | 0.75 | 5.0 | 1.3 | None |
| Example 2 | C2C12 | Temperature responsive dish | 45 | 0.75 | 5.0 | 1.3 | None |
| Example 3 | C2C12 | Temperature responsive dish | 135 | 0.75 | 5.0 | 1.3 | None |
| Example 4 | C2C12 | Temperature responsive dish | 40 | 0.75 | 5.0 | 1.3 | None |
| Example 5 | C2C12 | Temperature responsive dish | 140 | 0.75 | 5.0 | 1.3 | None |
| Example 6 | C2C12 | Temperature responsive dish | 30 | 0.75 | 5.0 | 1.3 | None |
| Example 7 | C2C12 | Temperature responsive dish | 150 | 0.75 | 5.0 | 1.3 | None |
| Example 8 | C2C12 | Temperature responsive dish | 25 | 0.75 | 5.0 | 1.3 | None |
| Example 9 | C2C12 | Temperature responsive dish | 155 | 0.75 | 5.0 | 1.3 | None |
| Example 10 | C2C12 | Temperature responsive dish | 90 | 0.75 | 1.3 | 1.3 | None |
| Example 11 | C2C12 | Temperature responsive dish | 90 | 0.75 | 5.0 | 1.3 | Ultrasonic waves generated |
| Example 12 | A549 | Temperature responsive dish | 90 | 0.75 | 5.0 | 1.3 | None |
| Example 13 | HEK293 | Temperature responsive dish | 90 | 0.20 | 5.0 | 0.70 | None |
| Example 14 | HUVEC | Temperature responsive dish | 90 | 0.20 | 5.0 | 0.70 | None |
| Example 15 | MDCK | Temperature responsive dish | 90 | 4.0 | 5.0 | 1.3 | None |
| Example 16 | hMSC | Temperature responsive dish | 90 | 0.75 | 5.0 | 1.3 | None |
| Example 17 | BAEC | Polystyrene dish | 90 | 4.0 | 5.0 | 1.3 | None |
| Comparative Example 1 | C2C12 | Temperature responsive dish | 0 | 0.75 | 5.0 | 1.3 | None |
| Comparative Example 2 | A549 | Temperature responsive dish | 0 | 0.75 | 5.0 | 1.3 | None |
| Comparative Example 3 | HEK293 | Temperature responsive dish | 0 | 0.20 | 5.0 | 0.70 | None |
| Comparative Example 4 | HUVEC | Temperature responsive dish | 0 | 0.20 | 5.0 | 0.70 | None |
| Comparative Example 5 | MDCK | Temperature responsive dish | 0 | 4.0 | 5.0 | 1.3 | None |
| Comparative Example 6 | hMSC | Temperature responsive dish | 0 | 0.75 | 5.0 | 1.3 | None |
| Comparative Example 7 | BAEC | Polystyrene dish | 0 | 4.0 | 5.0 | 1.3 | None |

**Table 2: Evaluation results of Examples 1 to 17 and Comparative Examples 1 to 7**

| | Sheet quality | Detachment time |
|---|---|---|
| Example 1 | A | A |
| Example 2 | A | A |
| Example 3 | A | A |
| Example 4 | B | B |
| Example 5 | B | B |
| Example 6 | B | B |
| Example 7 | B | B |
| Example 8 | C | C |
| Example 9 | C | C |
| Example 10 | B | C |
| Example 11 | A | AA |
| Example 12 | A | A |
| Example 13 | A | A |
| Example 14 | A | A |
| Example 15 | A | A |
| Example 16 | A | A |
| Example 17 | A | A |
| Comparative Example 1 | D | D |
| Comparative Example 2 | D | D |
| Comparative Example 3 | D | D |
| Comparative Example 4 | D | D |
| Comparative Example 5 | D | D |
| Comparative Example 6 | D | D |
| Comparative Example 7 | D | D |

According to the present disclosure, a cell detachment method that efficiently detaches a cell sheet while reducing tearing of the cell sheet can be provided. Furthermore, according to the present disclosure, a cell detachment system that efficiently detaches a cell sheet while reducing tearing of the cell sheet can be provided.

While the present disclosure has been described with reference to exemplary embodiments, it is to be understood that the disclosure is not limited to the disclosed exemplary embodiments. The scope of the following claims is to be accorded the broadest interpretation so as to encompass all such modifications and equivalent structures and functions.

## Claims

1. A cell detachment method that detaches a cell sheet adhering to a culture surface of a culture container (8) from the culture container (8) by using a cell detachment system, the method comprising:
a tapping process of tapping the culture container (8) in a first direction that contains a direction component parallel to the culture surface; and
a shaking process of shaking the culture container (8) in a second direction that contains a direction component parallel to the culture surface,
wherein the first direction and the second direction are different from each other as viewed in a direction perpendicular to the culture surface.

2. The cell detachment method according to Claim 1, wherein the first direction and the second direction form an angle 30° or more and 150° or less as viewed in the direction perpendicular to the culture surface.

3. The cell detachment method according to Claim 1 or 2, wherein the first direction and the second direction form an angle 45° or more and 135° or less as viewed in the direction perpendicular to the culture surface.

4. The cell detachment method according to any one of Claims 1 to 3, wherein the tapping process includes a process of periodically tapping the culture container (8).

5. The cell detachment method according to Claim 4, wherein, in the tapping process, the culture container (8) is tapped at a frequency greater than a frequency of shaking the culture container (8) in the shaking process.

6. The cell detachment method according to any one of Claims 1 to 5, further comprising a vibrating process of applying vibrations in an ultrasonic band to the culture container (8).

7. A cell detachment system that detaches a cell sheet adhering to a culture surface of a culture container (8) from the culture container (8), the cell detachment system comprising:
a tapping unit (3) that taps the culture container (8) in a first direction that contains a direction component parallel to the culture surface; and
a shaking unit (4) that shakes the culture container (8) in a second direction that contains a direction component parallel to the culture surface,
wherein the first direction and the second direction are different from each other as viewed in a direction perpendicular to the culture surface.
